# EUROPEAN PATENT APPLICATION

(11) **EP 1 615 034 A1**
(43) Date of publication of application: **11.01.2006**
(21) Application number: 04724403.3
(22) Date of filing: 30.03.2004
(51) Int. Cl.: G01N 33/547, G01N 33/53, C12M 1/40, C12M 1/34

(54) **FIXING AGENTS, METHOD OF FIXING SUBSTANCE WITH THE SAME, AND SUBSTRATE HAVING SUBSTANCE FIXED THERETO WITH THE SAME**

(30) Priority: 31.03.2003 JP 2003093834; 06.10.2003 JP 2003346560
(71) Applicant: KANAGAWA ACADEMY OF SCIENCE AND TECHNOLOGY, Kanagawa 213-0012 (JP); Taiko Denki Co, Ltd, Ota-ku Tokyo 146-8668 (JP)
(72) Inventor: ITO, Yoshihiro, Kawasaki-shi, Kanagawa 2130012 (JP); HASUDA, Hirokazu, Yokohama-shi, Kanagawa 2450061 (JP); KONNO, Tomohiro, 1560045 (JP); ISHIHARA, Kazuhiko, 1810011 (JP); YAMAUCHI, Tetsuya, Ota-ku, Tokyo 1460083 (JP)
(74) Representative: Cripps, Joanna Elizabeth
(86) International application number: PCT/JP2004/004510
(87) International publication number: WO 2004/088319

(57) **Abstract**

An agent for fixing (a) substance(s), which can fix various substances to be fixed on a substrate via covalent bonds and which has an excellent effect for the prevention of non-specific adsorption is disclosed. A polymer containing a plurality of phosphorylcholine groups and photoreactive groups in one molecule is used. The polymer binds to the substrate and to the substance to be fixed via the photoreactive groups, thereby the substance to be fixed is bound to the substrate via covalent bonds through the polymer, and non-specific adsorption is effectively prevented by the phosphorylcholine groups. Further, an agent for fixing (a) substance(s) for fixing the substance on a substrate, comprising a nonionic water-soluble macromolecule having at least 2 photoreactive groups in one molecule was also provided.

## Description

### Technical Field

The present invention relates to an agent for fixing (a) desired substance(s) such as polypeptides, nucleic acids lipids and the like on a substrate, a method for fixing (a) substance(s) using the same, and a substrate on which (a) substance(s) is(are) fixed by the same.

### Background Art

Immunoplates for immunoassays, on which antibodies or antigens arc fixed, DNA chips having chips on which nucleic acids are fixed, and the like, have been widely used. As one of the conventionally used methods for fixing proteins or nucleic acids on substrates, physical adsorption is widely used. That is, by bringing a hydrophobic substrate such as one made of polystyrene into contact with an aqueous solution of the protein or nucleic acid to be fixed, the protein or nucleic acid is fixed on the substrate by physical adsorption.

However, the method using physical adsorption has a drawback in that the binding between the substrate and the fixed substance is weak, so that the stability of the substrate on which the substance is fixed is insufficient. In addition, although blocking of the substrate with a protein (when fixing a protein) such as bovine serum albumin (BSA), cascin, skim milk or the like, or with a DNA (when fixing a DNA) such as salmon sperm DNA or the like is performed in order to prevent non-specific adsorption to the region not covered with the desired protein or nucleic acid, the effect for preventing non-specific adsorption by blocking is not necessarily satisfactory.

Fixation of proteins or nucleic acids to substrates is also carried out by covalently binding the functional groups on the substrate with the functional groups of the proteins or nucleic acids. However, by this method, in cases where the functional group to be used is located in the active site or in the vicinity thereof of the substance, the activity of the substance is lost by the fixation. Further, in cases where an appropriate functional group does not exist, the substance cannot be fixed by this method. Still further, although the non-specific binding is prevented by blocking as in the case of physical adsorption, the preventive effect is not necessarily satisfactory.

The references disclosing the above-mentioned prior art include the following:
Japanese Laid-open Patent Application (Kokai) No. 11-337551,
Japanese Laid-open Patent Application (Kokai) No. 2001-337089.

On the other hand, it is also known to fix a substance on a substrate using photoreactive groups (Y. Ito and M. Nogawa, "Preparation of a protein micro-array using a photo-reactive polymer for a cell adhesion assay," Biomaterials, 24, 3021-3026 (2003)). In this method, however, attention is not paid to the prevention of non-specific adsorption.

### Disclosure of the Invention

Accordingly, an object of the present invention is to provide an agent for fixing a substance, which can fix various substances to be fixed on a substrate via covalent bond, and which is excellent in the effect of prevention of non-specific adsorption, as well as to provide a method for fixing a substance using the same and to provide a substrate on which a substance is fixed using the same.

The present inventors intensively studied to discover that non-specific adsorption may be effectively prevented by utilizing a water-soluble polymer of which molecule is electrically neutral as a whole as an agent for binding a substance, thereby completing the present invention.

That is, the present invention provides an agent for fixing (a) substance(s), comprising a water-soluble polymer used for fixing a desired substance on a substrate, which water-soluble polymer has at least two photoreactive groups in one molecule, the molecule of the water-soluble polymer being electrically neutral as a whole.

The present invention also provides a method for fixing (a) substance(s) on a substrate, comprising coating the substrate with an aqueous solution or an aqueous suspension containing the substance to be fixed on the substrate and the agent for fixing (a) substance(s) according to the present invention; and irradiating the solution or suspension with light. The present invention further provides a substrate on which said substance was fixed by the method according to the present invention.

By the present invention, an agent for fixing (a) substance(s), which can fix various substances to be fixed on a substrate via covalent bond, and which is excellent in the effect of prevention of non-specific adsorption, as well as a method for fixing (a) substance(s) using the same and a substrate on which a substance is fixed using the same, was provided. According to the present invention, (a) substance(s) to be fixed may be fixed via covalcnt bonds irrespective of the type(s) of the substance(s), so that a stable fixed substrate may be obtained. Further, by fixing (a) substance(s) with the agent for fixing (a) substance(s) according to the present invention, non-specific binding is effectively prevented. Still further, when fixing (a) substance(s) using the agent for fixing (a) substance(s) according to the present invention, patterning of the fixed substance may be attained by carrying out selective irradiation, so that the substance may easily be fixed to an optional pattern such as microarray or the like.

### Best Mode for Carrying out the Invention

As mentioned above, the agent for fixing (a) substance(s) according to the present invention comprises a polymer having at least 2 photoreactive groups in one molecule, which molecule is electrically neutral as a whole. The term "molecule is electrically neutral as a whole" herein means that the molecule does not have a group which is ionized in aqueous solution having a pH in the vicinity of neutral (pH6-8), or even if the molecule has such groups, it has both of the groups which become cationic groups and which become anionic groups such that the total of the electric charges is substantially 0. The term "substantially" herein means that the total of the electric charges is 0, or even if the total is not 0, the total is small enough such that the electric charges do not adversely affect the present invention.

The agent for fixing (a) substance(s) according to the present invention is water-soluble, and the solubility to water (weight in terms of gram which can be dissolved in 100 g of water) is preferably not less than 5.

Preferred examples of the water-soluble polymer include the polymers containing the unit having the structure represented by the following general formula [I] and the unit having the structure represented by the following general formula [II]. 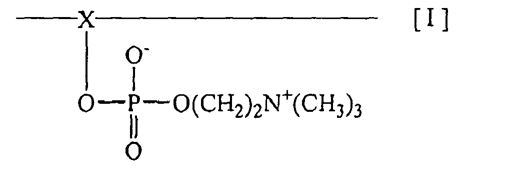 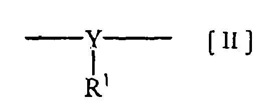 (wherein in general formulae [I] and [II], X and Y independently represent a polymerizable atomic group in the polymerized state, R¹ represents an atomic group having said photoreactive group, not less than 2 units represented by the general formula [I] and not less than 2 units represented by the general formula [II] are contained, and the number of the units represented by the general formula [I] is larger than the number of the units represented by the general formula [II].) (The unit having the structure represented by the above-described general formula [I] may be hereinafter referred to as "unit [I]" for convenience, and the unit having the structure represented by the above-described general formula [II] may be hereinafter referred to as "unit [II]" for convenience).

The above-described phosphorylcholine-containing polymer was invented based on the conception that non-specific adsorption may be effectively prevented by utilizing a macromolecule containing phosphorylcholine which is a constituent of biomembrane, paying attention to the fact that biomembranes hardly accompany non-specific adsorption in spite of the fact that biomembranes contact various substances.

The unit [I] is a unit containing phosphorylcholine group, and X represents a polymerizable atomic group in the polymerized state. As the X, vinyl monomer residues are preferred. As the unit [I], those represented by the following general formula [V] are preferred: 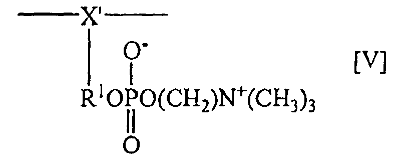 (wherein X' represents methacryloxy, methacrylamide, acryloxy, acrylamide, styryloxy or styrylamide group of which vinyl moiety is addition-polymerized, and R¹ represents single bond or C₁-C₁₀ alkylene (which may be substituted by 1 or 2 hydroxyl group)).

Preferred examples of such a unit include those derived from (i.e., those monomers in the polymerized state) 2-methacryloyloxyethylphosphorylcholine, 2-acryloyloxyethylphospholylcholine, N-(2-methacrylamide)ethylphospholylcholine 4-methacryloyloxybutylphospholylcholine, 6-methacryloyloxyhexylphospholylcholine, 10-methacryloyloxydecylphosphorylcholine, ω-methacryloyldioxyethylenephosphorylcholine and 4-styryloxybutylphosphorylcholine. Among these, the unit derived from 2-methacryloyloxyethylphosphorylcholine is most preferred.

On the other hand, a preferred example of the photoreactive group in the unit [II] is azide (-N₃) group, but the photoreactive group is not restricted thereto. As the Y in the unit of the general formula [II], vinyl monomer residues are preferred. Preferred examples of the unit [II] include those represented by the following general formula [VI]: 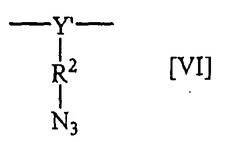 (wherein Y' represents methacryloxy, methacrylamide, acryloxy, acrylamide, styryloxy or styrylamide of which vinyl moiety is addition-polymerized; and R² represents single bond, C₁-C₁₀ alkylene (which may be substituted with 1 or 2 hydroxyl groups) or phenylene (which may be substituted with 1 to 3 substituents selected from C₁-C₄ alkyl and hydroxyl groups).

The number of unit [I] is larger than the number of unit [II]. Although the ratio is not restricted, the ratio is preferably about 100:2 to 100:50, more preferably about 100:5 to 100:20. Thus, since the polymer is mainly composed of the unit [I] having phosphorylcholine group, the non-specific adsorption is effectively prevented. The molecular weight of the water-soluble polymer is not restricted, and is usually about 1000 to 1,000,000, preferably about 5000 to 100,000.

Although the water-soluble polymer preferably consists of the unit [I] and unit [II] alone, it may contain units derived from other polymerizable monomers to the extent that the effect of the present invention is not adversely affected. Although the ratio of such other units is not restricted as long as the effect of the present invention is not adversely affected, the ratio is usually not more than 30 mol%, preferably not more than 10 mol% based on the total units in the polymer.

Preferred examples of the water-soluble polymer include those represented by the following general formula [III]: 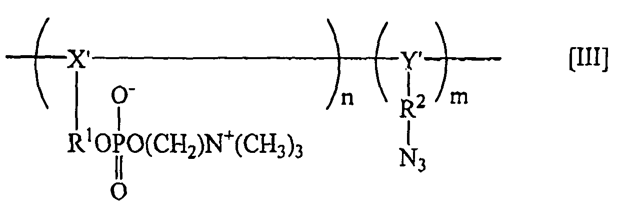 (wherein X', Y', R¹ and R² represent the same meanings as described above, respectively, n and m independently represent integers of not less than 2, and n is larger than m; the units containing X' and the units containing Y' are bound in a random order).

Among the compounds represented by the above-described general formula [III], the polymer represented by the following formula [IV] is especially preferred: 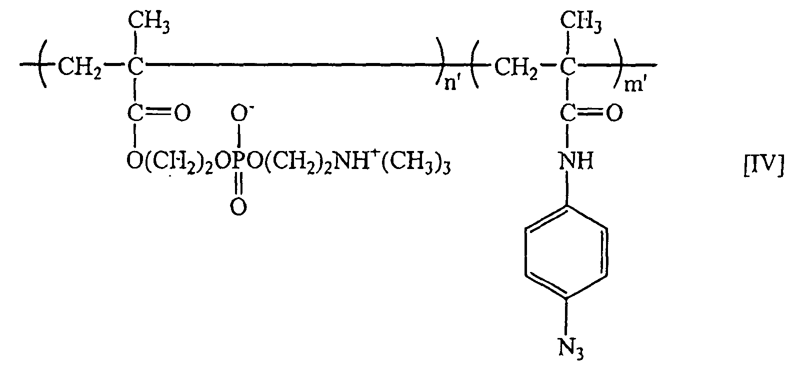 (wherein n' represents an integer of 50 to 200, m' represents an integer of 5 to 40, and the phosphorylcholine-containing units and the azidephenyl group-containing units are bound in a random order).

The above-described water-soluble polymer may be produced by simply polymerizing the above-described unit [I] and unit [II]. Alternatively, a polymer consisting of the main chain, which does not have the side chains (phosphorylcholine-containing groups and phosphoreactive group-containing groups) is first synthesized, and the side chains are bound later. Still alternatively, the unit [I] having the phosphorylcholine-containing group and the unit [II] which does not have the photoreactive group are first polymerized, and the photoreactive group-containing groups may be bound later. The Examples later described employ this method. The polymerization of the monomers and the binding of the side chains may be easily carried out by those skilled in the art according to common technical knowledge, and examples thereof are described in detail in the Examples below.

Preferred examples of the water-soluble polymer used in the present invention also include nonionic water-soluble macromolecule (polymer) having at least 2 photoreactive groups in one molecule. Nonionic water-soluble macromolecules have the effect for preventing non-specific adsorption comparable to the above-described phosphorylcholine-containing polymers, and have an advantage that they may be produced or available more inexpensively.

The term "nonionic" herein means that the molecule does not substantially have groups which are ionized in aqueous solution having a pH in the vicinity of neutral (pH6-8). The term "substantially" herein means that the molecule does not have such a group at all, or even if the molecule contains such a group, the amount thereof is so small (for example, the number of such groups is not more than 1% of the number of carbon atoms) that it does not adversely affect the present invention.

Although the molecular weight of the nonionic water-soluble macromolecule is not restricted, it is usually about 350 to 5,000,000, preferably about 500 to several hundred thousands.

Preferred examples of such a nonionic water-soluble macromolecule include polyalkylene glycols such as polyethylene glycol (PEG) and polypropylene glycol; nonionic vinyl macromolecules containing as the constituent the following monomers individually or in combination: vinyl alcohol, methylvinyl ether, vinylpyrrolidone, vinyloxazolidone, vinylmethyloxazolidone, 2-vinylpyridine, 4-vinylpyridine, N-vinylsuccinimide, N-vinylformamide, N-vinyl-N-methylformamide, N-vinylacetamide, N-vinyl-N-methylacetamide, 2-hydroxyethyl methacrylate, acrylamide, methacrylamide, N,N-dimethylacrylamide, N-*iso*-propylacrylamide, diacetoneacrylamide, methylolacrylamide, acryloylmorpholine, acryloylpyrrolidine, acryloylpiperizine, styrene, chloromethylstyrene, bromomethylstyrene, vinyl acetate, methyl methacrylate, butyl acrylate, methylcyano acrylate, ethylcyano acrylate, n-propylcyano acrylate, *iso*-propylcyano acrylate, *n*-butylcyano acrylate, *iso-*butylcyano acrylate, *tert*-butylcyano acrylate, glycidyl methacrylate, ethylvinyl ether, *n*-propylvinyl ether, *iso*-propylvinyl ether, *n*-butylvinyl ether, *iso*-butylvinyl ether and *tert*-butylvinyl ether; naturally occurring macromolecules such as gelatin, casein, collagen, gum arabic, xanthan gum, gum traganth, guar gum, pullulan, pectin, sodium alginate, hyaluronic acid, chitosan, chitin derivatives, carageenan, starches (carboxymethyl starch and aldehyde starch), dextrin and cyclodextrin; and naturally occurring macromolecules such as water-soluble cellulose derivatives including methyl cellulose, viscose, hydroxyethyl cellulose, hydroxyethylmethyl cellulose, carboxymethyl cellulose and hydroxypropyl cellulose; but the water-soluble nonionic macromolecules are not restricted thereto. Among these, especially preferred are polyethylene glycol, poly(meth)acrylamide (in the present Description and Claims, the term "(meth)acryl" means "methacryl" and "acryl") and poly(glycidyl (meth)acrylate), and polyethylene glycol is most preferred.

The above-described nonionic water-soluble macromolecules have at least 2 photoreactive groups per one molecule. Although the number of photoreactive groups per one molecule of the nonionic water-soluble macromolecule is not restricted as long as it is 2 or more, if the number is too large, there is a possibility that non-specific adsorption may be increased. Therefore, the number of the photoreactive groups per one molecule is preferably not more than 10%, more preferably, not more than 5% of the number of carbon atoms (excluding the carbons in the side chains) constituting the macromolecule. A preferred example of the photoreactive group is azide (-N₃) but the photoreactive group is not restricted thereto. Specific examples of the photoreactive group include phenyl azide, acetyl and benzoyl; and phenyl azide is especially preferred. Although the photoreactive group such as azide group may be directly bound to the nonionic water-soluble macromolecule, it may also be bound to the nonionic water-soluble macromolecule through an arbitrary spacer structure, the latter being usually easy to prepare and preferred. In the latter case, the spacer structure is not restricted at all, and examples of the spacer structure include C₁-C₁₀ alkylene (which may be substituted with 1 or 2 hydroxyl group) and phenylene (which may be substituted with 1 to 3 substituents selected from C₁-C₄ alkyl and hydroxyl groups).

Introduction of the photoreactive groups to the nonionic water-soluble macromolecule may easily be carried out based on a conventional method. For example, azide groups may be bound to a nonionic water-soluble macromolecule by reacting a nonionic water-soluble macromolecule having functional groups with an azide compound having a functional group which reacts with the functional groups of the nonionic water-soluble macromolecule. In cases where polyethylene glycol which is a preferred nonionic water-soluble macromolecule is used, since polyethylene glycols having amino groups or carboxyl groups at the both terminals thereof are commercially available, azide groups may be bound to the polyethylene glycol by reacting the functional groups of such a commercially available functional group-containing polyethylene glycol with an azide group-containing compound. A plurality of such methods are described in Examples below. Alternatively, in cases where the nonionic water-soluble macromolecule is one formed by polymerization of monomers, such as water-soluble vinyl macromolecules, a nonionic water-soluble macromolecule having photoreactive functional groups may be produced by copolymerizing a vinyl monomer which will constitute the main constituting units of the water-soluble vinyl macromolecule and a photoreactive vinyl monomer. Preferred examples of photoreactive water-soluble vinyl macromolecules include poly((meth)acrylamide-photoreactive(meth)acrylamide) copolymers, poly(glycidyl(meth)acrylate-photoreactive(meth)acrylamide) copolymers, poly(ethylene glycol mono(meth)acrylate-photoreactive acrylamide) copolymers and the like, and the method for producing these copolymers are described in detail in the Examples below.

The substance to be fixed by using the substance-fixing agent according to the present invention is not restricted, and examples thereof include polypeptides (including glycoproteins and lipoproteins), nucleic acids, lipids, cells (animal cells, plant cells, microorganism cells) and components of the cells (including nuclei, organelles such as mitochondria, membranes such as cell membranes and unit membranes and the like). When azide group which is used as the photoreactive group in the substance-fixing agent according to the present invention is irradiated with light, a nitrogen molecule is eliminated and simultaneously a nitrogen radical is generated. Since this nitrogen radical can bind with not only a functional group such as amino group or carboxyl group, but also with a carbon atom constituting an organic compound, the agent according to the present invention can fix most of the organic matters.

As the substrate, any substrate may be used as long as at least its surface is composed of a substance that can bind with the above-mentioned photoreactive group, and examples of the substrate include those made of polystyrene widely used in microplates and the like, polyethylene terephthalate, polycarbonate, polypropylene and the like. Glass plates coated with a silane coupling agent may also be used. The shape of the substrate is not restricted at all, and those in the form of plate such as substrates for microarrays, as well as those in the form of beads, fibers and the like, may be employed. Further, wells and grooves formed in a plate, such as the wells of microplates may also be used. Among these, the substance-fixing agent of the present invention is especially suited for microarrays.

Fixation of a desired substance on a substrate using the substance-fixing agent according to the present invention may be carried out as follows. First, an aqueous solution or an aqueous suspension containing the substance to be fixed on the substrate and the agent for fixing (a) substance(s) according to the present invention is applied on the substrate. In this case, although the concentration (by weight) of the substance-fixing agent in the aqueous solution is not restricted, usually it is about 0.005% to 10%, preferably about 0.04% to 1%. The concentration (by weight) of the substance to be fixed is usually about 10 to 200 times, preferably about 20 to 100 times of that of the substance-fixing agent used.

Thereafter, preferably after drying the applied liquid, light is radiated. The light is one which can make the photoreactive group used yield a radical, and in cases where azide group is employed as the photoreactive group, UV light is preferred. The dose of the light to be radiated is not restricted, and usually about 1 m W to 100 mW per 1 cm².

By irradiation with the light, the photoreactive groups in the polymer yield radicals, so that the polymer is bound with both the substrate and the substance to be fixed via covalent bonds. As a result, the substance to be fixed is fixed on the substrate through the polymer. Since the azide group used as the photoreactive group, upon being irradiated, eliminates a nitrogen molecule and simultaneously yields a nitrogen radical, and this nitrogen radical can bind with not only a functional group such as amino group or carboxyl group, but also with a carbon atom constituting an organic compound, the agent according to the present invention can fix most of the organic matters. By the method according to the present invention, since the binding reaction is carried out using the radicals generated from the photoreactive groups, the substance to be fixed is bound not at a particular site thereof, but at random sites. Therefore, although some molecules will naturally lose their activities because their active sites are used for the binding, there are molecules bound at sites which do not affect the active sites. Therefore, by the method of the present invention, even a substance which hitherto was difficult to be fixed via covalent bond because an appropriate functional group is located in the active site or a vicinity thereof may be fixed on the substrate via covalent bond without losing the activity thereof as a whole.

In the regions which were not irradiated with light, since the photoreactive groups binds with neither the substrate nor the substance, the polymer and the substance are removed by washing. Therefore, by carrying out selective exposure through a photo mask or the like, the substance may be fixed in an arbitrary pattern. Thus, the substance may be fixed in arbitrary various shapes such as microarray, which is very advantageous.

Alternatively, a mixture of the substance-fixing agent according to the present invention and the substance to be fixed is microspotted, and the entire surface of the substrate may be irradiated with light. Micro-spotting is a technique which applies liquid on very narrow regions on a substrate, and is widely used in the preparation of DNA chips and the like. Since apparatuses for conducting microspotting is commercially available, it may easily be carried out by using a commercially available apparatus. Alternatively, a method may be employed, in which the entire surface of the substrate is coated with the substance-fixing agent of the present invention, the substance to be fixed is microspotted thereon, and the entire of the substrate is irradiated with light. In this case, since spots of the substance to be fixed are formed on the layer of the substance-fixing agent, the ratio of the substance fixed on the substrate via covalent bond through the substance-fixing agent is increased. Still alternatively, a method may be employed, in which the substance-fixing agent of the present invention is microspotted on the substrate, the substance to be fixed is then microspotted on the microspots, and then the entire surface of the substrate is irradiated. In this case too, since spots of the substance to be fixed are formed on the layer (in the form of discrete spots) of the substance-fixing agent, the ratio of the substance fixed on the substrate via covalent bond through the substance-fixing agent is increased.

The method of the present invention may preferably be employed for the preparation of immunoassay plates on which antibodies or the antigen-binding fragments thereof, or antigens are fixed, nucleic acid chips and microarrays on which DNAs or RNAs are fixed, and the like, the applications of the method of the present invention are not restricted thereto. For example, it may be applied to the fixation of whole cells or components thereof.

The present invention will now be described more concretely by way of Examples. The present invention is not restricted to the Examples below.

### Example 1 Production of Substance-fixing Agent (Part 1)

According to the following synthesis scheme, a photoreactive methacryloyloxyethylphosphorylcholine (MPC) polymer was synthesized.

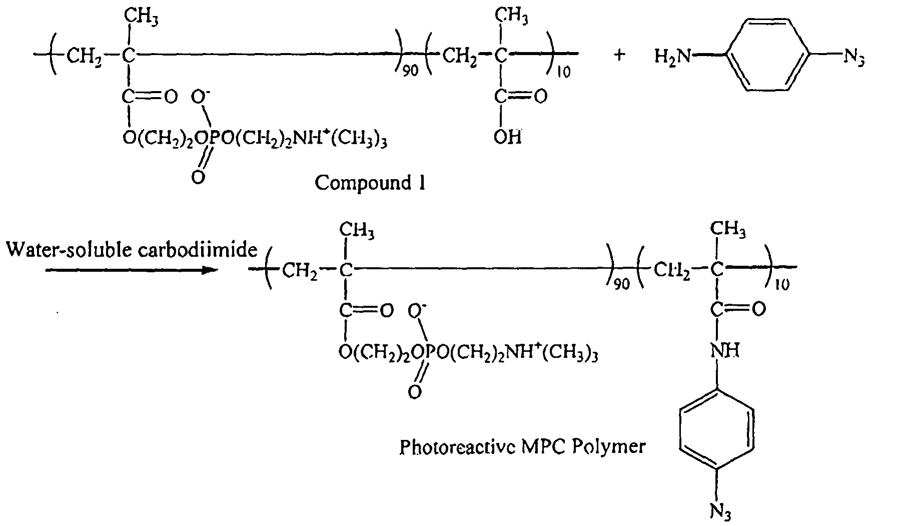

To 2 ml of aqueous PMAc solution (5wt%=50 mg/mL) (PMAc is the random copolymer of 2-methacryloyloxyethylphosphorylcholine (90%) and methacrylic acid (10%) and is the Compound 1 in the above-described reaction equation), 12.44 mg of 4-azideaniline and 17.47 mg of water-soluble carbodiimide (WSC) were added and then the total volume of the mixture was increased to 100 mL with pure water. The mixture was stirred at pH7 for 24 hours (at 4°C in a refrigerator). After completion of the reaction, the resulting mixture was subjected to dialysis using a dialysis cassette (produced by PIERCE) until the UV absorption of azideaniline in the external solution was no longer observed. Finally, the mixture was lyophilized to obtain photoreactive MPC polymer.

### Test Example 1 Non-specific Adsorption by Photoreactive MPC Polymer

Non-specific adsorption by the photoreactive MPC polymer prepared in Example 1 was tested as follows by bringing it into contact with FITC-conjugated protein or cells.

### (1) Binding of MPC polymer on Substrate

The photoreactive MPC polymer prepared in Example 1 was dissolved in pure water to a concentration of 10 mg/ml, and 25 µl aliquot thereof was applied on a polystyrene substrate (diameter: 2cm), followed by drying the solution in the air. Then UV (wavelength: 260 nm) was selectively radiated (irradiance: 16 mW/cm²) thereto through a photo mask having a stripe pattern with a width of 100 µm, and the substrate was then washed with water, thereby fixing the photoreactive MPC polymer on the polystyrene substrate.

### (2) Adsorption of FITC (fluorescein isothiocyanate)-conjugated Protein

On the substrate on which the above-described pattern was formed, 50 µl of an aqueous solution (concentration: 10 mg/ml) of FITC-BSA (commercially available from SIGMA), an aqueous solution (concentration: 2 mg/ml) of FITC-IgG (commercially available from SIGMA), or an aqueous FITC-fibrinogen solution (concentration: 3.7 mg/370 µl) was applied and the resultant was allowed to react at 37°C for 10 minutes. After washing the resulting substrate with PBS, the substrate was dried and observed with a fluorescence microscope.

As a result, fluorescence was observed only in the regions on which the photoreactive MPC polymer was not fixed, and very clear stripe pattern was observed. By this, it was confirmed that MPC polymer does not adsorb proteins non-specifically.

The FITC-fibrinogen used in the above-described test was prepared as follows. That is, to 20 mg of fibrinogen, 0.1 mg of FITC-I was added and the mixture was dissolved in 7 ml of 50 mM sodium carbonate buffer (pH8.5). The mixture was allowed to react overnight at 4°C and concentrated by centrifugation through a filter having an exclusion molecular weight of 3000. For desalination, the mixture was centrifuged 3 times with MilliQ (trademark) water, and then lyophilized to obtain 3.7 mg of FITC-fibrinogen.

### (3) Adsorption of Cells

The photoreactive MPC polymer prepared in Example 1 was fixed on a polystyrene substrate with the pattern as described above. After washing the substrate, the substrate was sterilized with 70% ethanol, and washed with sterilized water. The resulting substrate was incubated as it is, or after placing 250 µl of 1 % BSA/PBS solution or 1% fibrinogen/PBS solution on the substrate, at 37°C for 10 minutes. After washing the substrate with water, it was subjected to a test for adsorption of cells. The cells used were Cos 7 cells (commercially available from Cell Bank of RIKEN) and Raw 246 cells (commercially available from Cell Bank of RIKEN). The cells were scattered on the substrate and observed at 1 hour and 4 hours later with a microscope.

As a result, cells were scarcely adsorbed on the regions on which the photoreactive MPC polymer was fixed, so that the cells were adsorbed in the form of stripe. Particularly, clear stripe pattern was observed on the substrates on which photoreactive MPC polymer alone was applied and photoreactive MPC polymer plus fibrinogen were applied, respectively.

### Example 2

By the method described below, an immunoassay for detecting collagen using a collagen-fixed substrate prepared by the method of the present invention was carried out.
(i) Aqueous 0.1% solution of the photoreactive MPC polymer (prepared in Example 1) was mixed with aqueous 0.5% collagen solution at a ratio of 1:10.
(ii) On a 35 mm polystyrene dish, 3 µl of the MPC/collagen solution was spotted.
(iii) After drying, fixation was performed by irradiation with UV (wavelength: 260 nm, irradiance: 16 mW/cm², 10 seconds).
(iv) The substrate was washed 3 times with PBS.
(v) As a primary antibody, 100 µl of anti-collagen antibody (commercially available from MONOSAN) (5 µg/ml) or anti-humm CD3 antibody (commercially available from PHARMINGEN) was spotted, and the resultant was incubated at room temperature for 4 hours.
(vi) After washing the resultant 3 times with PBS, 100 µl of FITC-labeled secondary antibody (anti-rabbit IgG antibody or anti-mouse IgG antibody) (commercially available from AMERSHAM) (5000-fold diluted), and the resultant was incubated at room temperature for 1 hour.
(vii) After washing the resultant 3 times with PBS, the substrate was observed with a fluorescence microscope.
(viii) As controls, the same procedures described above were repeated except that MPC polymer alone was spotted, or except that the collagen solution alone was spotted on a Hubble slide glass produced by TAKARA. Further, as controls, the same procedures described above were repeated except that an aqueous solution of azidephenyl group-introduced polyacrylic acid (PAAc) having negative charge only and collagen, or an aqueous solution of azidephenyl group-introduced polyallylamine (PAAm) having positive charge only and collagen, was spotted on the polystyrene substrate.

### Results and Discussion

After the immunostaining with the anti-collagen antibody, fluorescence was observed on the MPC polymer/collagen spots with a fluorescence microscope and the spots were not stained with the anti-CD3 antibody, so that it was confirmed that collagen was stained specifically. In the controls, substantially no fluorescence was observed on the spots of MPC polymer alone, and no fluorescence was observed on the spots of azidephenyl group-introduced polyacrylic acid (PAAc) having negative charge alone and collagen. On the spots of azidephenyl group-introduced polyallylamine (PAAm) having the positive charge only, non-specific staining occurred irrespective of the type of the antibody. From the above, the property of MPC polymer that non-specifc adsorption is small was experimentally proved. Although fixation of collagen on the Hubble slide glass produced by TAKARA was tried as a control, fluorescence was not observed on the spots, and it is thought that collagen could not be fixed in this case. From the above, it was proved that proteins can be fixed via covalent bonds using MPC polymer, the non-specific adsorption is inhibited, and so the use of MPC polymer is effective. The results are summarized in Table 1 below.

**Table 1**

| | Anti-collagen Antibody | Anti-CD3 Antibody |
|---|---|---|
| MPC polymer/collagen | fluorescence observed | no fluorescence |
| MPC polymer | no fluorescence | no fluorescence |
| PAAc/collagen | no fluorescence | no fluorescence |
| PAAm/collagen | fluorescence observed | fluorescence observed |

### Example 3 Production of Substance-fixing Agent (Part 2)

### (1) Synthesis of N-(4-azidebenzoyloxy)succinimide

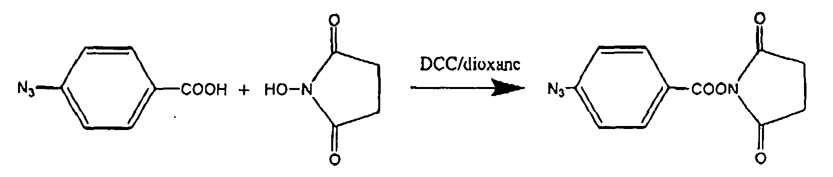

In 40 ml of 1,4-dioxane, 600 mg of 4-azidebenzoic acid (commercially available from TOKYO KASEI) and 420 mg of N-hydroxysuccinimide (commercially available from WAKO PURE CHEMICALS) were dissolved, and 760 mg of N,N'-dicyclohexylcarbodiimide (commercially available from WAKO PURE CHEMICALS) was added, followed by stirring the resulting mixture at room temperature to start the reaction. Twenty four hours later, by-product was removed by filtration through a filter paper, and the solvent of the filtrate was evaporated under reduced pressure. The obtained product was purified by recrystallization twice from 1,4-dioxane and diethyl ether, to obtain 675 mg of N-(4-azidebenzoyloxy)succinimide (yield: 70.6%).

### (2) Introduction of Azide Group into Polyethylene Glycol

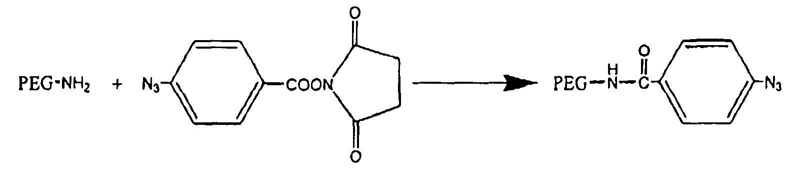

In 10 ml of dimethylformamide (commercially available from WAKO PURE CHEMICALS), 100 mg of poly(ethylene glycol)bis(3-aminopropyl) terminals (commercially available from ALDRICH, average molecular weight: 1500, hereinafter also referred to as "PEG-NH₂" for convenience) and 68.6 mg of the N-(4-azidebenzyloxy)succinimide prepared in (1) were dissolved, and the mixture was stirred at pH6 at 4°C for 24 hours. After completion of the reaction, DMP was evaporated under reduced pressure with an evaporator, and pure water was added to precipitate the unreacted products. The precipitates were sedimented by centrifugation and the supernatant was recovered, followed by lyophilization thereof to obtain photoreactive PEG-NH₂ (Although the NH₂ groups no longer exist because they were consumed by the binding with the azide compound, the product is referred to such for convenience).

### Example 4 Production of Substance-fixing Agent (Part 3)

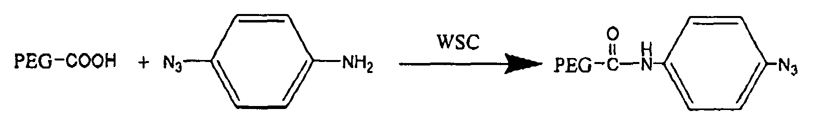

In 10 ml of pure water, 100 mg of poly(ethylene glycol)bis(carboxymethyl) ether (commercially available from ALDRICH, average molecular weight: 600, hereinafter referred to as "PEG-COOH" for convenience), 113.3 mg of 4-azideaniline and 127 mg of water-soluble carbodiimide were dissolved, and the solution was stirred at pH6 at 4°C for 24 hours. After completion of the reaction, the pH was adjusted to 12 with sodium hydroxide to precipitate the unreacted products. After sedimenting the precipitates by centrifugation, the supernatant was recovered and desalted with chloroform using a separation funnel (this operation was repeated until the pH became neutral), followed by lyophilization of the product to obtain photoreactive PEG-COOH (Although the COOH groups no longer exist because they were consumed by the binding with the azide compound, the product is referred to such for convenience).

### Example 5 Production of Substance-fixing Agent (Part 4)

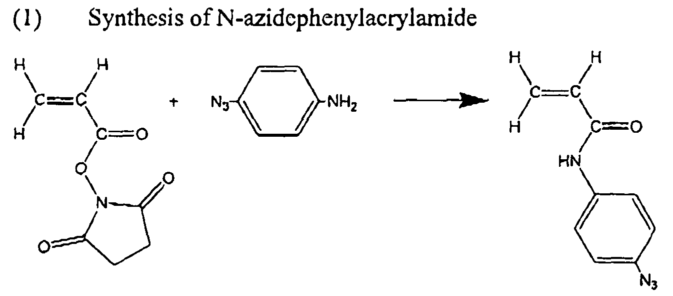

In 10 ml of DMF, 341.2 mg of 4-azideaniline (commercially available from ALDRICH) and 169.1 mg of N-acryloxysuccinimide (commercially available from ALDRICH) were dissolved, and the mixture was stirred at 4°C to start the reaction. Twenty four hours later, the product was purified by recrystallization to obtain N-azidcphenylacrylamide.

### (2) Synthesis of Photoreactive Poly(acrylamide-N-azidephenylacrylamide) Copolymer

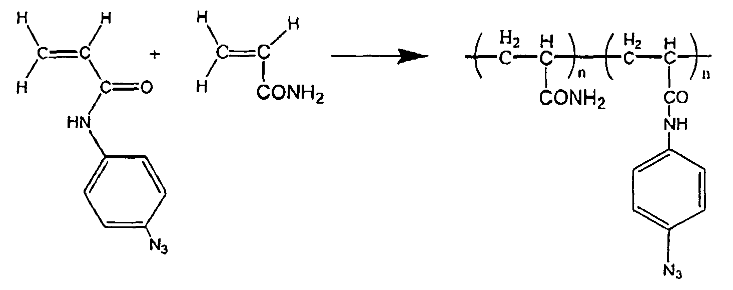

In 30 ml of ethanol, 639.7 mg of acrylamide (commercially available from WAKO PURE CHEMICALS) and 160 mg of N-azidephenylacrylamide were dissolved, and 82.1 mg of 2,2'-azobisisobutyronitrile (commercially available from WAKO PURE CHEMICALS) as a polymerization initiator was added, followed by stirring the mixture at 60°C to start the reaction. Five hours later, the polymer was purified by reprecipitation in acetone and the resultant was dried under reduced pressure to obtain photorcactive poly(acrylamide-N-azidephenylacrylamide) copolymer.

### Example 6 Production of Substance-fixing Agent (Part 5)

### Synthesis of Photoreactive Poly(glycidyl methacrylate-N-azidephcnylacrylamide) Copolymer

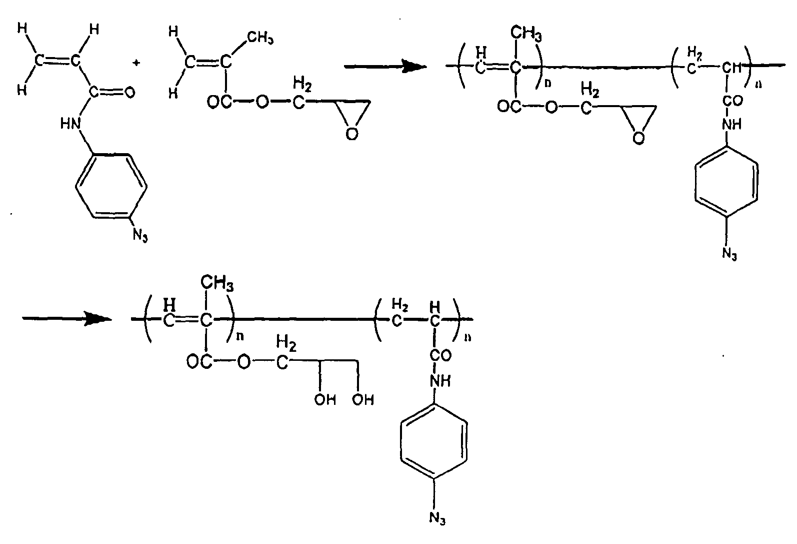

In 30 ml of methanol, 1.28 g of glycidyl methacrylate (commercially available from ALDRICH) and 160 mg of N-azidephenylacrylamide were dissolved, and 16.42 mg of 2,2'-azobisisobutyronitrile as a polymerization initiator was added, followed by stirring the mixture at 60°C to start the reaction. Five hours later, the polymer was purified by reprecipitation in diethyl ether and the product was dried under reduced pressure to obtain photoreactive poly(glycidyl methacrylate-N-azidephenylacrylamide) copolymer. The epoxy groups were converted to glycerol by a treatment with sodium hydroxide (pH12) to make the product water-soluble. Comparative Example 1 Production of Photoreactive Polyacrylic Acid

Photoreactive polyacrylic acid was prepared as follows: In 100 ml of pure water, 720 mg of polyacrylic acid (commercially available from WAKO PURE CHEMICALS, average molecular weight: 1,000,000), 170.6 mg of 4-azideaniline and 1.917 g of water-soluble carbodiimide were dissolved, and the resulting solution was stirred at pH7.0 at 4°C for 24 hours. After completion of the reaction, the resulting mixture was subjected to dialysis until the UV absorption of azidcaniline in the external solution was no longer observed. Finally, the mixture was lyophilized to obtain photoreactive polyacrylic acid polymer.

### Comparative Example 2 Production of Photoreactive Polyallylamine

The same procedures as in Comparative Example 1 were repeated except that the polyacrylamine was used in place of polyacrylic acid to produce photoreactive polyacrylamine.

### Example 7 Immunoassay

### (1) Preparation of Collagen-fixed Substrate and Immunoassay of Anti-collagen Antibody Using the Same

By the procedures described below, using the substance-fixing agents prepared in Examples 3 to 6, Example I, Comparative Example 1 and Comparative Example 2, respectively, collagen-fixed substrates (polystyrene dish) were prepared, and immunoassays of anti-collagen antibody were carried out using these substrates.
(i) The photoreactive poly(acrylamide-N-azidephenylacrylamide) copolymer (Example 5) or photoreactive poly(glycidyl methacrylate-N-azidephenylacrylamide) copolymer (Example 6) (0.125 wt%) and collagen (0.25 wt%) were mixed at a ratio of 1:1.
(ii) On a 35 mm polystyrene dish, 0.5 µl of the aqueous polymer/collagen solution was spotted.
(iii) After drying, fixation was performed by UV irradiation (wavelength: 260 nm, irradiance: 40 mW/cm², 10 seconds).
(iv) The substrate was washed 3 times with PBS (0.1 % Tween 20).
(v) As a primary antibody, 100 µl of 10-fold diluted solution of anti-collagen antibody (commercially available from MONOSAN) solution (1% BSA/PBS) or 40-fold diluted solution of mouse IgG antibody (commercially available from SANTA CRUZ BIOTECHNOLOGY) was applied in the form of spots, and the resultant was incubated at room temperature for 3 hours.
(vi) After washing the resultant 3 times with PBS (0.1 % Tween 20 (trademark)), 100 µl of 20-fold diluted solution of FITC-labeled secondary antibody (anti-rabbit IgG antibody or mouse IgG antibody) (commercially available from AMERSHAM) was added, and the resultant was incubated at room temperature for 1 hour.
(vii) After washing the resultant 3 times with PBS (0.1 % Tween 20 (trademark)), the substrate was observed with a fluorescence microscope.
(viii) The same procedures were repeated except that a substrate on which the photoreactive MPC polymer (Example 1), the photoreactive polyacrylic acid for comparison (Comparative Example 1), photoreactive polyallylamine (Comparative Example 2) or the substrate on which the substance-fixing agent alone was spotted prepared in each Example was used.

### (2) Results

As shown in Table 2 below, after immunostaining with anti-collagen antibody, fluorescence was observed on PEG-NH₂ (Example 3)/collagen spots, PEG-COOH (Example 4)/collagen spots, poly(acrylamide-N-azidephenylacrylamide) copolymer (Example 5)/collagen spots, poly(glycidyl methacrylate-N-azidephenylacrylamide) copolymer (Example 6)/collagen spots, and fluorescence was not observed on the anti-IgG antibody spots. By this, it was confirmed that collagen was stained specifically. With the PEG-NH₂ (Example 3) and PEG-COOH (Example 4), the fluorescence intensity was about the same as that observed on the MPC polymer/collagen spots (Example 1) stained with the anti-collagen antibody, and the fluorescence intensity observed on the poly(acrylamide-N-azidephenylacrylamide) copolymer spots (Example 5) and poly(glycidyl methacrylate-N-azidephenylacrylamide) copolymer spots (Example 6) was about 1/2 thereof. In contrast, on the photoreactive polyacrylic acid having negative charge only/collagen spots (Comparative Example 1), there was no fluorescence, and on the photoreactive allylamine having positive charge only/collagen spots (Comparative Example 2), non-specific staining occurred irrespective of the type of the antibody. By these results, it was proved that photoreactive polyethylene glycol can fix proteins to the degree comparable to the MPC polymer, and can inhibit non-specific adsorption. With the poly(acrylamide-N-azidephenylacrylamide) copolymer (Example 5) and with the poly(glycidyl methacrylate-N-azidephenylacrylamide) copolymer (Example 6), fixation can be attained, although the amount of the fixed protein is smaller than that attained by using polyethylene glycol (Examples 3 and 4), and non-specific adsorption is inhibited.

**Table 2**

| Substance-fixing Agent | Collagen | Fluorescence Intensity | |
|---|---|---|---|
| | | Anti-collagen Antibody | Anti-IgG Antibody |
| Example 3 | used | 932 | 25.5 |
| | not used | 28 | 29 |
| Example 4 | used | 1483 | 55.5 |
| | not used | 62 | 63 |
| Example 5 | used | 583 | 33 |
| | not used | 26 | 28 |
| Example 6 | used | 602 | 29 |
| | not used | 30 | 28 |
| Example 1 | used | 1185 | 33 |
| | not used | 30 | 31 |
| Comparative Example 1 | used | 156 | 39 |
| | not used | 44 | 27 |
| Comparative Example 2 | used | 82 | 95 |
| | not used | 79 | 90 |

### Example 8 Production of Substance-fixing Agent (Part 6)

### 1. Synthesis of N-azidephenylacrylamide

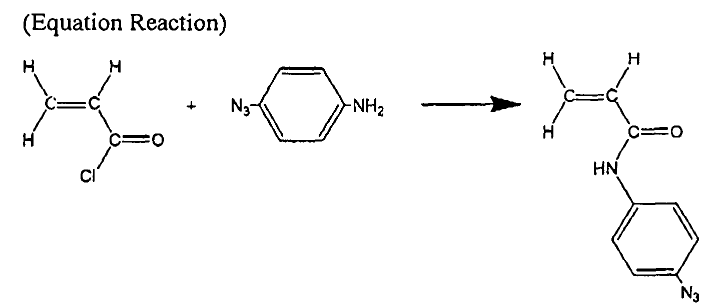

In 97 ml of pure water, 300.02 mg of 4-azideaniline hydrochloric acid salt (commercially available from ALDRICH) was dissolved, and 3 ml of aqueous 2 mol/l NaOH solution was added thereto. To the solution, 200 mg of acrylic acid chloride ((commercially available from TOKYO KASEI) diluted with dichloromethane to a volume of 13 ml was slowly dropped while stirring the mixture, and after the dropping, the mixture was vigorously agitated after tightly stopping the vessel. The precipitated solids were recovered by filtration, and the dichloromethane phase was separated after adding 80 ml of chloroform. The separated organic layer was dried and the solids were recovered. As a result, N-azidephenylacrylamide was obtained.

### 2. Synthesis of Photoreactive Poly(ethylene glycol monomethacrylate-N-azidephenylacrylamide) Copolymer

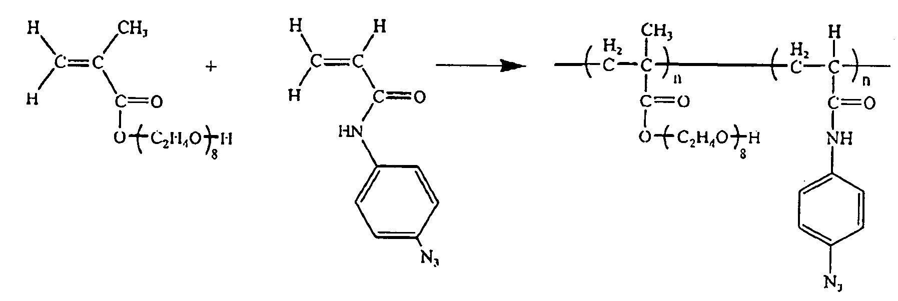

In a 300 ml eggplant type flask, 49.90 mg of N-azidephenylacrylamide and 2225.2 mg of PEG (polymerization degree n=8) were dissolved in 43 ml of ethyl acetate, and 6.31 mg of azobisisobutyronitrile (AIBN) was added thereto. The flask was filled with nitrogen and was tightly stopped, followed by stirring in water bath at 60°C for 6 hours. After stopping the heating and after evaporation of the solvent to some degree, the mixture was transferred to diethyl ether, and the resulting mixture was subjected to sonicator for 30 minutes. The solids were recovered and dried under reduced pressure for 2 hours. As a result, photoreactive poly(ethylene glycol monomethacrylate-N-azidephenylacrylamide) copolymer was obtained.

### Example 9 Preparation of Allergen-fixed Substrates and Immunoassays of Anti-allergen Antibodies Using the Same

Cedar allergen or mite allergen (commercially available from SEIKAGAKU CORPORATION) was dissolved in pure water to a concentration of 0.25 wt%, and the solution was mixed with the photoreactive polymer (0.125 wt%) prepared in Example 1 or Example 8 at a mixing ratio of 1:1 (v/v). On a polystyrene substrate, 0.5 µl of the mixed allergen/polymer solution was spotted. Then the substrate was irradiated with UV (wavelength: 260 nm, irradiance: 15 mW/cm²) to fix the allergen on the polystyrene substrate.

The substrate was then washed with PBS (0.1 % Tween 20), and 100 µl of anti-allergen antibody (commercially available from SEIKAGAKU CORPORATION) as the primary antibody dissolved in human serum to a level of 100 µg/ml was placed in the form of spots on the substrate, followed by allowing the resultant to react at room temperature for 2 hours. The substrate was then washed with PBS (0.1% Tween 20), and 10 µl of HRP-labeled secondary antibody (commercially available from AMERSHAM BIOSCIENCE) 100-fold diluted with 10% BSA/PBS was added on the spots, followed by allowing the resultant to react at room temperature for 1 hour. After washing the substrate with PBS (0.1 % Tween 20), 10 µl of a chemiluminescence reagent ECL ADVANCE (commercially available from AMERSHAM BIOSCIENCE) was added, and exposed for 30 seconds with a gel photographing system to obtain an image. The obtained image was digitized by an analysis software.

The digitized luminescence intensities are summarized in Table 3 below.

The one which exhibited the highest luminescence intensity at an antibody level of 100 ng/ml was PEG and the copolymer of Example 8. The one which exhibited low luminescence intensity when the antibody level was 0 was also the copolymer of Example 8, so that it was shown that the non-specific adsorption thereof was small.

By the above-described results, it was shown that the copolymer of Example 8, by which the fixed amounts of the allergens were large and the non-specific adsorption was small, is best suited as the matrix used for allergen arrays.

**Table 3**

| | Luminescence Intensity | | | |
|---|---|---|---|---|
| | Anti-cedar Allergen | | Anti-Mite | Allergen |
| | Antibody Level (ng/ml) | | Antibody Level (ng/ml) | |
| | 0 | 100 | 0 | 100 |
| PEG | 4086 | 22963 | 1986 | 19921 |
| Acrylamide | 3193 | 12357 | 10156 | 21350 |
| Example 1 (MPC) | 6829 | 23348 | 0 | 1567 |
| Example 8 | 1357 | 19517 | 798 | 22096 |
| Allergen alone | 0 | 8593 | 0 | 213 |

### Industrial Availability

The present invention relates to an agent for fixing (a) substance(s) by which a substance to be fixed on a substrate can be fixed via covalent bond, and which is excellent in the prevention of non-specific adsorption, as well as to a method for fixing (a) substance(s) using the same and to the substrate on which the substance was fixed using the same. According to the present invention, (a) substance(s) to be fixed may be fixed via covalent bonds irrespective of the type(s) of the substance(s), so that a stable fixed substrate may be obtained. Further, by fixing (a) substance(s) with the agent for fixing (a) substance(s) according to the present invention, non-specific binding is effectively prevented. Still further, when fixing (a) substance(s) using the agent for fixing (a) substance(s) according to the present invention, patterning of the fixed substance may be attained by carrying out selective irradiation, so that the substance may easily be fixed to an optional pattern such as microarray or the like. Therefore, the present invention is useful for the preparation of various microarrays and the like.

## Claims

1. An agent for fixing (a) substance(s), comprising a water-soluble polymer used for fixing a desired substance on a substrate, which water-soluble polymer has at least two photoreactive groups in one molecule, said molecule of said water-soluble polymer being electrically neutral as a whole.

2. The agent for fixing (a) substance(s) according to claim 1, comprising a unit having the structure represented by the following general formula [I] and a unit having the structure represented by the following general fonnula [II]: 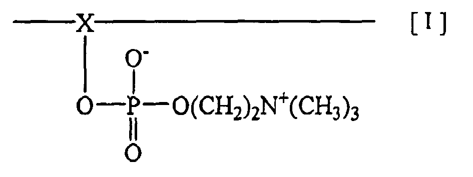 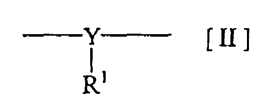 (wherein in general formulae [I] and [II], X and Y independently represent a polymerizable atomic group in the polymerized state, R¹ represents an atomic group having said photoreactive group, not less than 2 units represented by the general formula [I] and not less than 2 units represented by the general formula [II] are contained, and the number of the units represented by the general formula [I] is larger than the number of the units represented by the general formula [II].)

3. The agent for fixing (a) substance(s) according to claim 1 or 2, wherein said photoreactive group is azide group.

4. The agent for fixing (a) substance(s) according to claim 2 or 3, wherein the ratio of the number of said units represented by the general formula [I] to the number of said units represented by the general formula [II] is 100:2 to 100:50.

5. The agent for fixing (a) substance(s) according to any one of claims 1 to 4, wherein said polymer has a molecular weight of 1000 to 1,000,000.

6. The agent for fixing (a) substance(s) according to any one of claims 1 to 5, wherein said X and Y are derived from vinyl monomers.

7. The agent for fixing (a) substance(s) according to any one of claims 1 to 3, which is represented by the following general formula [III]: 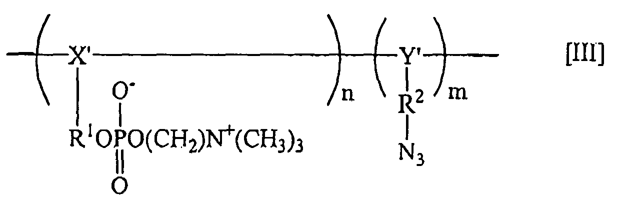 (wherein X' and Y' independently represent methacryloxy, methacrylamide, acryloxy, acrylamide, styryloxy or styrylamide group, in the state in which the vinyl moiety thereof is addition-polymerized; R¹ represents a single bond or C₁-C₁₀ alkylene (with the proviso that it may be substituted with 1 or 2 hydroxyl groups), R² represents a single bond or C₁-C₁₀ alkylene (with the proviso that it may be substituted with I or 2 hydroxyl groups) or phenylene group (with the proviso that it may be substituted with 1 to 3 substituents selected from C₁-C₄ alkyl and hydroxyl groups); n and m independently represent integers of not less than 2, and n is larger than m; the units containing X' and the units containing Y' are bound in a random order).

8. The agent for fixing (a) substance(s) according to claim 7, wherein said unit containing X' is derived from 2-methacryloyloxyethylphospholylcholine, 2-acryloyloxyethylphospholylcholine, N-(2-methacrylamide)ethylphospholylcholine, 4-methacryloyloxybutylphospholylcholine, 6-methacryloyloxyhexylphospholylcholine, 10-methacryloyloxydecylphosphorylcholine, ω-methacryloyldioxyethylenephosphorylcholine or 4-styryloxybutylphosphorylcholine.

9. The agent for fixing (a) substance(s) according to claim 8, wherein said unit represented by the general formula [I] is derived from 2-methacryloyloxyethylphosphorylcholine.

10. The agent for fixing (a) substance(s) according to claim 9, which is represented by the following formula [IV]: 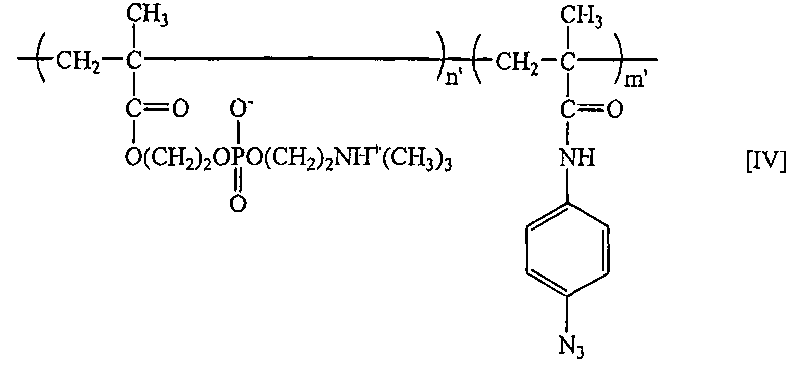 (wherein n' represents an integer of 50 to 200, m' represents an integer of 5 to 40, and the phosphorylcholine-containing units and the azidephenyl group-containing units are bound in a random order).

11. The agent for fixing (a) substance(s) according to any one of claims 1 to 10, wherein said substance to be fixed on said substrate is selected from the group consisting of polypeptides, nucleic aids, lipids and cells and constituents of the cells.

12. A method for fixing (a) substance(s) on a substrate, comprising coating said substrate with an aqueous solution or an aqueous suspension containing said substance(s) to be fixed on said substrate and said agent for fixing (a) substance(s) according to any one of claims 1 to 11; and irradiating said solution or suspension with light.

13. The method according to claim 12, wherein said substance(s) to be fixed on said substrate is(are) selected from the group consisting of polypeptides, nucleic aids, lipids, cells and constituents of the cells.

14. The method according to claim 12 or 13, comprising selectively radiating said light so as to pattern the region to which said substance is fixed.

15. A substrate on which said substance(s) was(were) fixed by the method according to any one of claims 12 to 14.

16. The agent for fixing (a) substance(s) according to claim 1, wherein said water-soluble polymer is a nonionic water-soluble macromolecule having at least 2 photoreactive groups in one molecule.

17. The agent for fixing (a) substance(s) according to claim 16, wherein said nonionic water-soluble macromolecule is a polyalkylene glycol, polyvinyl macromolecule or a naturally occurring macromolecule.

18. The agent for fixing (a) substance(s) according to claim 17, wherein said polyalkylene glycol is polyethylene glycol, said polyvinyl macromolecule is poly((meth)acrylamide-photoreactive(meth)acrylamide) copolymer or poly(glycidyl(meth)acrylate-photoreactive(meth)acrylamide) copolymer.

19. The agent for fixing (a) substance(s) according to claim 18, wherein said nonionic water-soluble macromolecule is polyethylene glycol, poly(acrylamide-photoreactive acrylamide) copolymer or poly(glycidyl methacrylate-photoreactive acrylamide) copolymer.

20. The agent for fixing (a) substance(s) according to any one of claims 16 to 19, wherein said photoreactive group is phenyl azide group.

21. The agent for fixing (a) substance(s) according to any one of claims 16 to 20, wherein said nonionic water-soluble macromolecule has a molecular weight of 500 to 5,000,000.

22. The agent for fixing (a) substance(s) according to any one of claims 16 to 20, wherein said substance(s) to be fixed on said substrate is(are) selected from the group consisting of polypeptides, polysaccharides, nucleic aids, lipids, cells and constituents of the cells.

23. A method for fixing (a) substance(s) on a substrate, comprising coating said substrate with an aqueous solution or an aqueous suspension containing said substance(s) to be fixed on said substrate and said agent for fixing (a) substance(s) according to any one of claims 16 to 22; and irradiating said solution or suspension with light.

24. The method according to claim 23, wherein said substance(s) to be fixed on said substrate is(are) selected from the group consisting of polypeptides, polysaccharides, nucleic aids, lipids and cells and constituents of the cells.

25. A substrate on which said substance(s) was(were) fixed by the method according to any one of claims 16 to 24.
